Europäisches Patentamt

European Patent Office

Office européen des brevets

(19)

(11) Numéro de publication: **0 154 609**
**A1**

(12)

# DEMANDE DE BREVET EUROPEEN

(21) Numéro de dépôt: **85830014.8**

(22) Date de dépôt: **24.01.85**

(51) Int. Cl.⁴: **A 61 F 5/02**

(30) Priorité: **06.02.84 IT 1153884 U**

(43) Date de publication de la demande:
**11.09.85 Bulletin 85/37**

(84) Etats contractants désignés:
**AT BE CH DE FR GB LI LU NL SE**

(71) Demandeur: **CO.PRO.SA. S.r.l.**
**Viale Italia 210**
**I-19100 La Spezia(IT)**

(72) Inventeur: **Bellati, Patrizia**
**Viale Italia 210**
**I-19100 - La Spezia(IT)**

(74) Mandataire: **Martini, Lazzaro**
**Ufficio Brevetti Ing. Lazzaro Martini Via Brunelleschi, 1**
**I-50123 Firenze(IT)**

(54) Appareil pneumatique d'élongation pour le traitement des déformations de la colonne vertébrale.

(57) Un appareil d'élongation pour le traitement des déformations de la colonne vertébrale comprenant une plaque (1) en matériel rigide avec une côte (2) également rigide, se dressant sur une des faces de la plaque et ayant la forme d'un huit allongé, le sommet duquel est fermé par une membrane (3) élastique, à même de délimiter deux chambres à air (30) cylindriques et communicant entre elles au moyen d'un étroit conduit (20).

Lesdites chambres (30) peuvent être gonflées au moyen d'un pompe (5) manuelle et l'appareil est propre à l'emploi avec un corset placé autour du tronc du patient.

Fig. 2

EP 0 154 609 A1

"Appareil pneumatique d'élongation pour le traitement des déformations de la colonne vertébrale"

La présente invention concerne un appareil pneumatique d'élongation pour le traitement des déformations de la colonne vertébrale.

On sait que la colonne vertébrale n'est pas rectiligne, mais présente une série d'incurvations très prononcées et discontinues selon le plan vertical. Les premières, correspondant chacune à une portion de la colonne vertébrale, sont au nombre de quatre, c'est-à-dire les cambrures cervicales et lombaires, convexes vers l'avant, et les cambrures dorsales et sacrées, convexes vers l'arrière. Des déviations de la colonne vertébrale provoquent diverses situations pathologiques comme le dos rond, l'hyperlordose lombaire, la scoliose, etc.

On sait que ces déformations de la colonne vertébrale ont jusqu'à présent été corrigées, selon leur gravité, au moyen de corsets et de bustes orthopédiques, de plâtres, d'inverventions chirurgicales de stabilisation vertébrale comme l'arthorodèse. A l'exception des plâtres et des interventions chirurgicales qui sont réservés aux cas les plus graves, les corsets et les bustes orthopédiques connus actuellement se servent de plaques ou d'attelles en métal ou en plastique rigide, mais de tels tuteurs sont peu efficaces et provoquent souvent chez le patient de graves malaises physiques et psychiques.

La présente invention a pour but d'éviter ces incon
vénients en proposant un dispositif caplable de cor
riger, de manière non sanglante, les déformations
de la colonne vertébrale.

On est parvenu à ce résultat en conformité avec l'
invention, telle qu'elle est caractérisée dans les
revendications, en adoptant l'idée suivante, consis
stant à provoquer une élongation longitudinale de
la zone déformée de colonne vertébrale, jusqu'à ce
que soit rétablie la cambrure correcte de la colonne, en utilisant la poussée verticale divergente de
deux vessies élastiques, espacées mais unies entre
elles par un petit tube et gonflées avec de l'air
et tenues en place par un corset ordinaire de tissu

Les avantages de la présente invention consistent
essentiellement en ce que la poussée divergente d'
elongation est localisable et réglable en intensité; en ce qu'il est possible d'obtenir des démodifi
cations stables et permanentes des diverses déforma
tions de la colonne vertébrale; en ce que le dispositif est de fabrication facile et d'application
simple et aisée et en ce qu'il est bien toléré, me-
me après de longues périodes.

L'invention est exposée ci-après plus en détail à
l'aide de dessins représentant seulement un mode d'
execution.
La figure 1 représente la vue en section verticale
d'un appareil pneumatique d'elongation selon l'invention dans une éventuelle position d'utilisation;
la figure 2 représente la vue axonométrique d'ensem

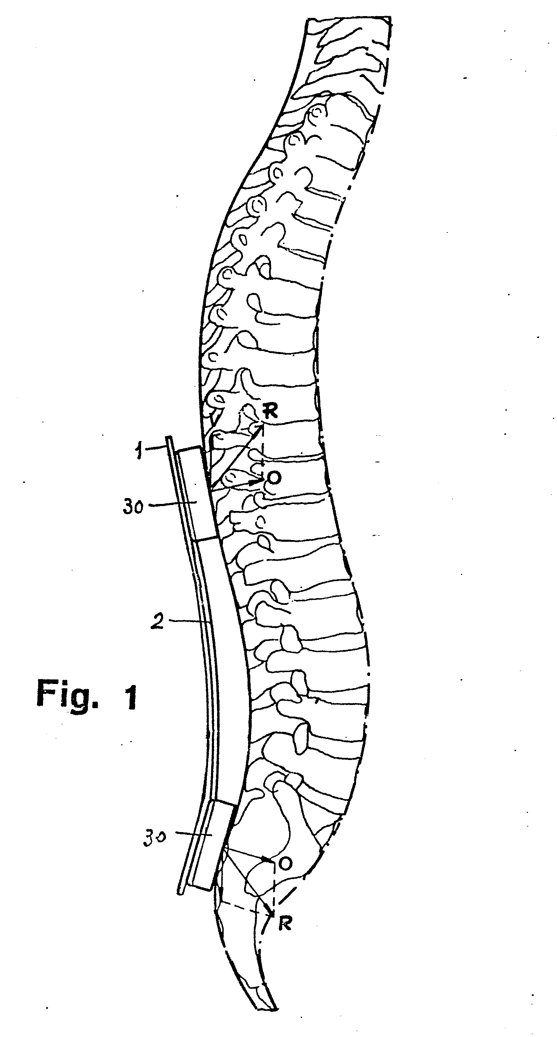

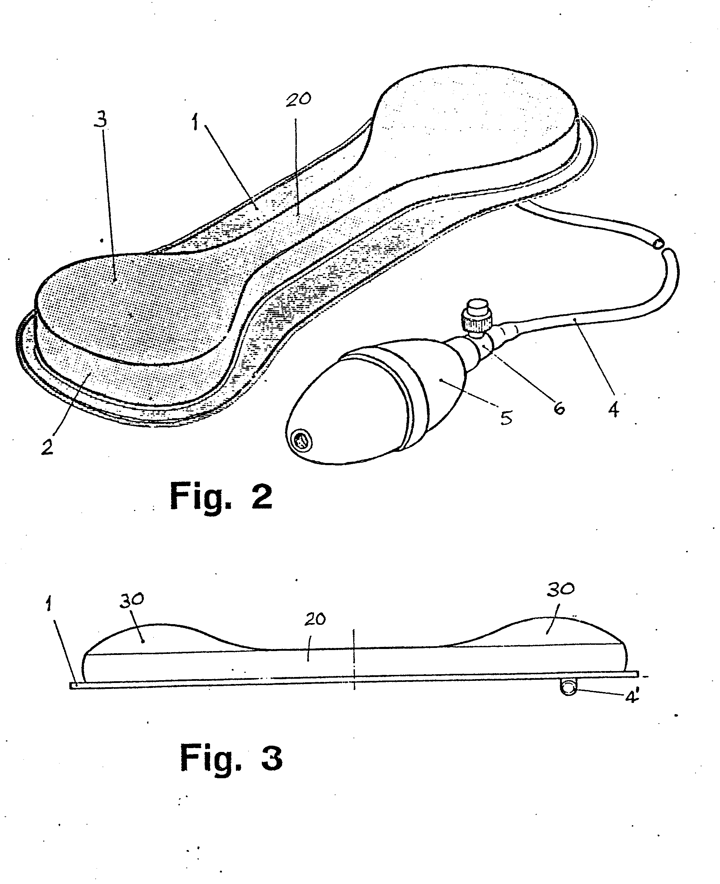

ble de l'appareil d'élongation de la figure 1 en position de repos; la figure 3 représente la vue latérale de l'appareil d'élongation de la figure 1 en position gonflée, mais non utilisé. .

Un appareil pneumatique d'élongation pour le traitement des déformations de la colonne vertébrale conforme à l'invention, est composé:

- d'une plaque 1 sur une face de laquelle se dresse une côte 2 qui prend la forme d'un huit allongé et fermé dans sa partie supérieure par une membrane 3 flexible et élastique laquelle définit avec ladite côte 2 et ladite plaque 1 deux chambres à air 30 et un conduit central 20 qui rend communicantes les deux chambres à air 30. Une des deux chambres à air 30 est pourvue d'une prise d'air, passant de préférence à travers ladite plaque 1, et pourvue d'une bride pour permettre d'attacher un tube de caoutchouc 4,qui est à son tour relié à une pompe manuelle 5 avec soupape 6 de rétention et de dégonflage.

L'appareil d'élongation est préalablement placé, non gonflé, à l'intérieur d'un corset ordinaire de tissu, avec les deux chambres à air 30 tournées vers et placées contre le dos du patient, en position verticale et avec la chambre à air inférieure placée en correspondance avec le passage lombo-sacré, tandis que la chambre à air supérieure se trouve mise en correspondance avec la zone de la colonne à traiter et est maintenu dans cette position par les lacets serrés du corset.

Ensuite, actionnant la pompe 5, les chambres à air 30 se gonflent et leurs membranes 3, sous la pres-

- 4 -

sion de l'air se redressent en acquérant une certaine dureté tout en conservant une conformation essentiellement plane, ce à quoi contribue également la côte 2. L'accroissement en hauteur ou en épaisseur des chambres à air 30 a lieu en direction de et contre le dos du porteur étant donné que la plaque 1 est rigide et partant indéformable, de sorte qu'une force R est exercée par chaque chambre à air 30 sur la zone correspondante d'appui. Les deux forces R, qui sont diversement orientées, c'est-à-dire vers le haut pour ce qui en est de celle provenant de la chambre à air supérieure 30 et vers le bas en ce qui concerne celle de la chambre à air inférieure, ont donc leurs respectives composantes verticales V, ou plus exactement parallèles aux respectives surfaces d'appui, qui sont de sens contraire. Ces deux forces V exercent une traction longitudinale sur la partie de colonne vertébrale comprise entre les deux chambres à air 30 de l'appareil d'élongation et cette traction, même en suffisante pour corriger les cambrures irrégulières de la colonne. Il est évident que les forces horizontales O, ou plus exactement les forces perpendiculaires à la surface d'appui des chambres à air, finissent par être annulées par la contrepression exercée par le corset.

## R E V E N D I C A T I O N S

1) Un appareil d'élongation pour le traitement des déformations de la colonne vertébrale, caractérisé par le fait que cet appareil est composé d'une plaque (1) en plastique rigide, avec une côte (2) se dressant sur une des faces de la plaque, ayant la forme d'un huit allongé et sur le sommet duquel se trouve une membrane (3) flexible et élastique, à même de délimiter, avec la côte (2) et la plaque (1), deux chambres à air (30) et un étroit conduit central (20) de communication entre lesdites chambres à air (30).

2) Un appareil d'élongation selon la revendication 1) caractérisée par le fait qu'une des chambres à air (30) est pourvue d'une prise d'air (4) passant à travers ladite plaque, avec une bride permettant sa fixation à un tube en caoutchouc (4).

3) Un appareil d'élongation selon la revendication 2) caractérisée par le fait que ledit tube (4) est relié à une pompe manuelle (5) avec une soupape (6) de rétention et de dégonflage.

Fig. 1

**Fig. 2**

**Fig. 3**

## DOCUMENTS CONSIDERES COMME PERTINENTS

| Catégorie | Citation du document avec indication, en cas de besoin des parties pertinentes | Revendication concernée | CLASSEMENT DE LA DEMANDE (Int Cl 4) |
|---|---|---|---|
| X | GB-A-2 031 732 (N.A. ROMANO)<br>* Page 3, lignes 62-102; page 4, lignes 16-24; figures 1,4-6 * | 1-3 | A 61 F 5/02 |
| A | US-A-2 427 546 (C.W. BROOKS)<br>* Colonne 1, lignes 23-34; figures 1,2 * | 1 | |
| A | EP-A-0 099 783 (ARLUX)<br>* Page 5, lignes 4-9; page 6, lignes 1-18; figure 4 * | 1-3 | |
| A | EP-A-0 077 760 (M. GIONTELLA)<br>* Page 3, lignes 9-23; page 2, lignes 15-20; figure 2 * | 1-3 | |
| A | FR-E- 8 857 (L.M.C. CHARNAUX)<br>* Page 1, lignes 26-34, lignes 40-45; page 2, lignes 7-12; figure 4 * | 1 | **DOMAINES TECHNIQUES RECHERCHES (Int Cl 4)**<br><br>A 61 F |
| A | FR-A-2 338 033 (INSERM)<br>* Page 4, lignes 21-26; page 5, ligne 6 * | 1,2 | |
| A | GB-A- 244 042 (A. KLOTZ)<br>* Page 1, lignes 39-65; figures 1,2 * | 1 | |
|   | --- -/- | | |

Le present rapport de recherche a ete etabli pour toutes les revendications

| Lieu de la recherche<br>LA HAYE | Date d'achévement de la recherche<br>15-05-1985 | Examinateur<br>WOLF C.H.S. |
|---|---|---|

**Office européen des brevets**

## RAPPORT DE RECHERCHE EUROPEENNE

| DOCUMENTS CONSIDERES COMME PERTINENTS | | | |
|---|---|---|---|
| Catégorie | Citation du document avec indication. en cas de besoin des parties pertinentes | Revendication concernee | CLASSEMENT DE LA DEMANDE (Int Cl 4) |
| A | US-A-4 178 923 (J.D. CURLEE)<br>* Colonne 4, lignes 5-15; lignes 38-57 *<br><br>--- | 1-3 | |
| A | CH-A- 403 159 (C.L.A. GODEBERGE)<br>* Page 1, ligne 59 - page 2, ligne 39; figures 1,6,9 *<br><br>--- | 1 | |
| A | GB-A- 320 884 (H. COLQUHOUN et al.)<br>* Page 2, lignes 2,3, 24-31; figure 3 *<br><br>----- | 1-3 | |

DOMAINES TECHNIQUES RECHERCHES (Int Cl 4)

Le present rapport de recherche a ete etabli pour toutes les revendications

| Lieu de la recherche | Date d achevement de la recherche | Examinateur |
|---|---|---|
| LA HAYE | 15-05-1985 | WOLF C.H.S. |